# EUROPÄISCHE PATENTSCHRIFT

(11) **EP 1 082 971 B1**
(45) Veröffentlichungstag und Bekanntmachung des Hinweises auf die Patenterteilung: **12.04.2006**
(21) Anmeldenummer: 00118725.1
(22) Anmeldetag: 30.08.2000
(51) Int. Cl.: A61M 15/00

(54) **Spender für Medien**
Fluid dispenser
Distributeur de fluides

(30) Priorität: 08.09.1999 DE 19942791
(43) Veröffentlichungstag der Anmeldung: 14.03.2001
(73) Patentinhaber: Ing. Erich Pfeiffer GmbH, 78315 Radolfzell (DE)
(72) Erfinder: Ritsche, Stefan, 78315 Radolfzell (DE)
(74) Vertreter: Patentanwälte Ruff, Wilhelm, Beier, Dauster & Partner

(56) Entgegenhaltungen:
- WO-A-94/22510
- WO-A-99/31952
- DE-A- 19 704 849
- DE-A- 19 750 872
- DE-A- 19 831 525
- US-A- 5 284 132
- DATABASE WPI Week 199804 Derwent Publications Ltd., London, GB; AN 1998-041699 XP002164765 & WO 97 41831 A (CORNELL RES FOUND INC), 13. November 1997 (1997-11-13)

## Beschreibung

Die Erfindung betrifft einen Spender für Medien nach dem Oberbegriff des Anspruchs 1. Diese können flüssig, gasförmig, fest oder bevorzugt pulverförmig sein. Der Spender soll einhändig zu tragen und gleichzeitig mit derselben Hand zu betätigen sein. Ferner sollen seine Bauteile teilweise oder vollständig aus Kunststoff bzw. durch Spritzguß hergestellt sein. Das fließfähige Medium kann durch Saugen bzw. Einatmen gefördert werden. Dabei wird der pharmazeutische Wirkstoff des Mediums inhaliert oder auf der Nasenschleimheit abgelagert.

Die WO 94/22510 beschreibt einen Spender, der eine Luftpumpe und einen in den Luftpumpenzylinder einsetzbaren, in diesem beweglichen Medienspeicher enthält. In dem Pumpzylinder läuft ein Kolben, der in eine an der Außenseite des Zylinders geführte Hülse eingesetzt ist. Im Inneren des Kolbens ist, ebenso wie am Austrittskanal, je ein Stößel angebracht, die den Speicher nach seiner Verschiebung auf die Austrittsöffnung zu an beiden Seiten durch Durchstoßen einer Versiegelung öffnet Eine Betätigungshandhabe als Fingerauflage ist als ein extra Ring aufgeschoben .

Die US 5 284 132 zeigt einen Spender mit zwei ineinander schiebbaren, zylindrischen Grundkörpern, die jeweils Fingerauflagen haben. Von der Mitte eines der G rundkörper ragt ein Pumpenkolben in den anderen G rundkörper hinein. Die seitlichen Fingerauflagen für Zeige- und Mittelfinger sind als gerade seitliche Auskragungen ausgebildet. Der Erfindung liegt die Aufgabe zugrunde, einen Spender zu schaffen, bei welchem Nachteile bekannter Ausbildungen vermieden sind und der insbesondere bei einfachem Aufbau sehr kompakt ist. Ferner soll der Spender sehr handlich und bequem zu betätigen sein. Der Spender soll gegen Beschädigungen durch axiale oder radiale Druckbelastungen geschützt sein. Das Gewicht des Spenders soll sehr gering sein. Des weiteren soll der Spender einfach herzustellen bzw. zu montieren sein.

Diese Aufgabe wird dadurch gelöst, dass die Kompaktlagerung drei Gehäusemäntel aufweist, von denen zwei unter Bildung eines Laufmantels und eines Führungsmantels zusammenwirken und der weitere Führungsmantel in einer Halterung einen wenigstens eine Austragdosis des Mediums im wesentlichen dicht aufnehmenden Speicher umgibt, dass beide Führungsmäntel an dem ersten Grundkörper angeformt sind und der Laufmantel an dem zweiten Grundkörper, dass der Laufmantel der Kompaktlagerung am Außenumfang von einem Außenmantel des zweiten Grundkörpers umgeben ist und dass eine den Außenmantel und den Laufmantel verbindende Endwand des zweiten Grundkörpers eine Betätigungshandhabe der zweiten Einheit bildet.

Ein in Strömungsrichtung frei ausragender Mantel kann frei zugänglich an der Außenseite des Spenders liegen und zur Führung der stromabwärtigen Spendereinheit dienen, die einen an der Innenseite dieses Führungsmantels gleitenden Laufmantel haben kann. Greift der Laufmantel zwischen zwei gleichgerichtete Führungsmäntel unmittelbar ein, so können diese auch entgegen Strömungsrichtung frei ausragen und an der zweiten Spendereinheit vorgesehen sein. Dadurch ist eine sehr exakte Führung und ein labyrinthartig abgedichteter Verschluß des Innenraumes des Spenders geschaffen. Außerdem können Arretierglieder für die beiden Einheiten im Spalt zwischen den Führungsmänteln bzw. im Spalt zwischen einem Führungsmantel und dem Laufmantel geschützt liegen.

Die Aufnahme oder Halterung für den Speicher kann vollständig innerhalb eines Mantels liegen, in welchen dann die Speicherschale berührungsfrei hineinragt. Alle drei Gehäuse-Mäntel können so die Speicherkammer permanent vollständig umgeben und gegen Verformungen durch äußere Druckbelastungen schützen. Die Arretierung kann verhindern, daß die Einheiten axial auseinandergezogen und getrennt werden oder daß die Einheiten gegeneinander verdreht werden. Dabei kann die Anordnung so sein, daß in einer Drehstellung das Abziehen unter Überwindung einer federnden Schnappkraft erleichtert und in einer weiteren Drehstellung gegen wesentlich höhere Abzugkräfte verhindert bzw. nur durch Zerstörung möglich ist. Die erste bzw. die zweite Einheit kann in ihren Querschnitten zentrischsymmetrisch ausgebildet sein.

Davon abweichend kann die zweite Einheit voneinander weggerichtete Queransätze aufweisen, die als einzige Bereiche der zweiten Einheit radial über den Außenumfang der ersten Einheit vorstehen und zur Abstützung jeweils eines Fingers bei der manuellen Betätigung des Spenders, nämlich dessen Verkürzung, dienen. Der Laufmantel kann an die Innenseite dieser Queransätze einteilig unmittelbar anschließen und eine geringfügig erweiterte Fortsetzung eines äußersten Stutzenmantels der zweiten Einheit bilden. Innerhalb dieses Stutzenmantels liegt ein Innenmantel, welcher einen Dorn zur Öffnung des Speicherverschlusses beim Arbeitshub bilden und den Auslaßkanal sowie gemeinsam mit dem äußeren Stutzenmantel den Medienauslaß begrenzen kann.

Der Spender kann aus nur zwei Bauteilen bestehen, von denen jeder einteilig ist und in die dann nur noch der Speicher, beispielsweise ein Blister, einzusetzen ist. Auch eine lösbare Sperre gegen unbeabsichtigtes Betätigen kann einteilig mit einer dieser Einheiten ausgebildet sein und die andere Einheit durch Anschlag an einer Stirnfläche sperren. Das Sperrglied wird entweder geschwenkt oder abgerissen, um die Betätigung des Arbeitshubes freizugeben. Auch eine entsprechend hohe Betätigungskraft für den Arbeitshub kann das Sperrglied ausrücken und so eine hohe Spannkraft zu Beginn des Arbeitshubes bewirken.

Der Spender ist insbesondere zum Austrag biologischer Wirkstoffe, wie biochemischer Hilfsstoffe geeignete, welche die Arzneistoffwirkung der pharmazeutischen Wirkstoffe steuern bzw. unterstützen können, z.B. durch Verzögerung der Arzneistofffreisetzung, durch Verbesserung der Resorption o. dgl., ohne selbst eine unmittelbar pharmakologische Wirkung haben zu müssen. Die Stoffe können aber auch Peptide, Proteine und/oder Hormone sein, z.B. Steroidhormone, Polypeptid- oder Proteohormone, Releasing-Hormone, Oxytocin, Vasopressin, Insulin, Glucagon, Parathormon, Calcitonin, Schilddrüsenhormone, Katecholamine, Acetylcholin, Prostaglandine o. dgl. Des weiteren ist der Spender zum Austrag von Adjuvantien, wie eines Freundschen Adjuvans, geeignet, welche bei gemeinsamer Applikation mit einem Antigen die Antwort des Immunsystems unspezifisch verstärken bzw. die Art der Immunantwort verändem und Aluminiumverbindungen, Mineralöle oder inaktivierte Mykobakterien enthalten können. Solche pharmakokinetischen Arzneimittel können auch die Wirkung eines anderen Heilmittels unterstützen.

Diese und weitere Merkmale der Erfindung gehen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein und vorteilhafte Ausführungen darstellen können. Ausführungsbeispiele der Erfindung sind in den Zeichnungen dargestellt und werden im folgenden näher erläutert. In den Zeichnungen zeigen:
- Fig. 1: einen erfindungsgemäßen Spender in teilweise geschnittener Ansicht,
- Fig. 2: die erste Einheit des Spenders gemäß Fig. 1 in Draufsicht,
- Fig. 3: die zweite Einheit des Spenders in Ansicht von unten, und
- Fig. 4: einen Ausschnitt der Fig. 1 in verdrehter und vergrößerter Darstellung.

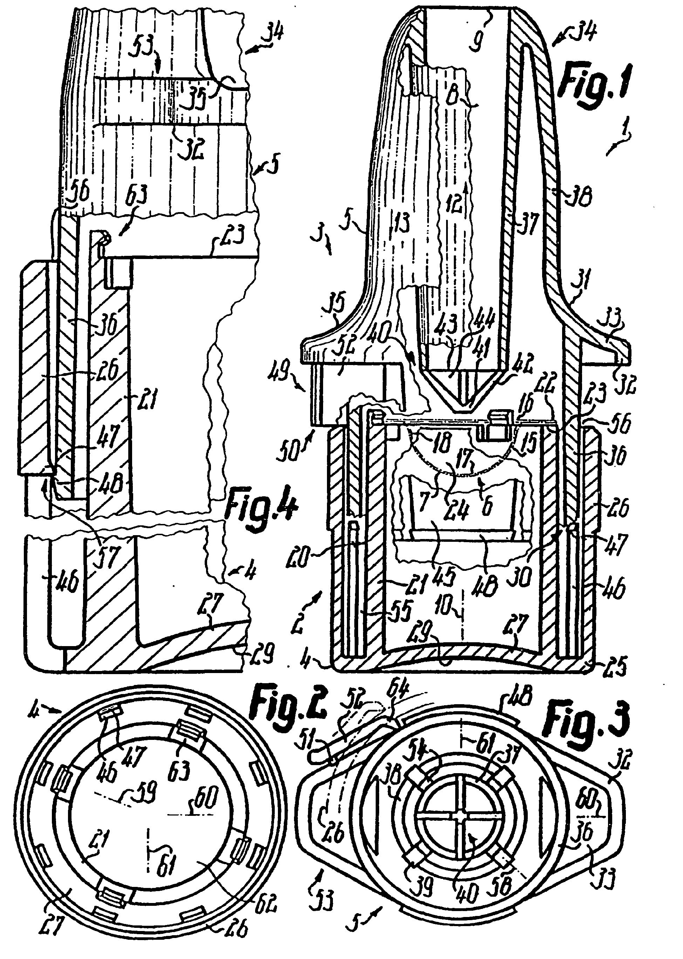

Der Spender 1 hat in der Ruhelag gemäß Fig. 1 eine Länge von höchstens 70 mm oder 55 mm und eine nur höchstens halb so große Diametralausdehnung seiner zwei Einheiten 2, 3. Die Einheiten 2, 3 werden unter Verkürzung des Spenders und ohne Relativdrehung über einen Arbeitshub in eine betätigte Endlage überführt. Die Einheiten 2, 3 können ohne simultane Längenänderung gegeneinander über mehr oder weniger als 360° verdreht werden bzw. gegen Verdrehen aneinander gesichert sein. Die erste Einheit 2 besteht ausschließlich aus einem einteiligen Grundkörper 2 sowie ggf. einem in diesen eingesetzten Speicherkörper 6. Die zweite Einheit 3 besteht ausschließlich aus einem einteiligen Grundkörper 5, dessen Gesamtlänge mindestens drei Fünftel der Länge des Spenders 1 entspricht. Der Spender 1 dient zur Aufnahme einer einzigen Mediendosis und kann nach deren Austrag recycled werden. Die Mediendosis ist wesentlich kleiner als das Speichervolumen. Beim Austrag strömt das Medium durch den Speicher 7 unmittelbar in einen geradlinigen Auslaßkanal 8 und aus dessen Ende bzw. Medienauslaß 9, dessen Durchmesser größer als 3 mm oder 5 mm ist. Alle genannten Teile liegen permanent in einer gemeinsamen Spenderachse 10, zu welcher die Austragrichtung 12 und die entgegengesetzte Betätigungsrichtung 13 parallel liegen. Der Medienauslaß kann auch eine Zerstäuberdüse sein.

Der Speicher 7 hat einen Speichernapf 15, dessen ebene Schalenöffnung mit einem Speicherverschluß 16, wie einer ebenen Membran oder Folie, hermetisch verschlossen ist. Der halbkugelförmige Schalenboden 17 geht in einen Speichermantel 18 über, welcher an einen ebenen, ringförmigen Speicherrand 22 anschließt.

Der Speicherkörper 6 hat keine Durchbrüche und liegt vollständig innerhalb des Tragkörpers 20 der Einheit 2. Die Körper 4, 5 bilden eine Kompaktlagerung und weisen Gehäuse-Mäntel 21, 26, 36, nämlich zwei Führungsmäntel 21, 26 am Körper 4 und einen dazwischen liegenden Laufmantel 36 am Körper 5, auf. Der innere Führungsmantel 21 bildet den Tragkörper 20, an dessen freier Endfläche 23 der biegeflexible oder elastische Rand 22 abgestützt ist.

Der Körper 4 ist eine gegenüber seinem Durchmesser längere Kappe, deren äußerster und frei zugänglicher Mantel der äußere Führungsmantel 26 ist. Beide Mäntel 21, 22 stehen in Richtung 12 frei von einer End- oder Stirnwand 27 ab, deren zentrisch vertiefte Außenseite eine Betätigungshandhabe 29 bildet. Die Schale 15 liegt mit Radialabstand innerhalb des Mantels 21, ohne daß die Wandungen 17, 18 zusätzlich abgestützt sind. Die Schulter 23 steht über den Mantel 26 geringfügig vor.

Beim Einsetzen des Körpers 6 wird eine formschlüssige Sicherung 63 wirksam, welche über die Schulter 23 vorstehende, federnde Schnappvorsprünge 63 aufweist, die den Rand 22 gegen die Schulter 23 drücken, ohne daß der Speicherkörper 6 formschlüssig verdrehgesichert ist.

Die Mäntel 21, 26, 36 bilden die einzige sowie nach außen vollständig abgedeckte Verbindung 30 zwischen den Körpern 4, 5. Auch der Körper 5 ist eine Kappe, welche symmetrisch zu zwei zueinander rechtwinkligen Axialebenen 60, 61 ist und in der Ebene 60 die größere Radialausdehnung hat. Der Kappenmantel 36 steht von der Stirnwand 33 in Richtung 13 frei vor und hat eine Dicke, welche größer als der halbe Abstand zwischen den Mänteln 21, 26 ist, so daß der Mantel 36 gleichzeitig oder wahlweise an beiden Mänteln 21, 26 gleitend geführt ist. Die freie Endfläche des Mantels 26 schlägt am Hubende am freien Ende eines Außenmantels 32 an, welcher radial außerhalb des Mantels 36 in Richtung 13 von der Stirnwand 33 absteht.

In dieser Endstellung sind zwischen den einander zugekehrten Umfangsflächen der Mäntel 21, 26, 36 Kanalwege 55 frei. Sie bilden Ansaugkanäle für Außenluft mit labyrinthartig entgegengesetzten Strömungsrichtungen und reichen über den gesamten Umfang der Körper 4, 5. Die Einlaßöffnungen 56 können am freien Ende des Mantels 26 liegen und ringförmig sein oder durch Durchbrüche 46 im Mantel 26 gebildet sein. Die Luft strömt zwischen den Mänteln 21, 36 in Richtung 12 zur Platte 22, von dort radial zur Achse 10 und dann in Richtung 13 in die Kammer 24, von wo sie unter Mitnahme des Mediums zurück in Richtung 13 unmittelbar aus der Öffnung 9 austritt. Durch manuelles Abdecken der Ansaugöffnungen 46 kann die Luftströmung bei gleicher Saugleistung variabel gedrosselt werden.

Die Mäntel 32, 36 und die Stirnwand 33 gehen in einen in Richtung 12 frei vorstehenden Auslaßstutzen 34 über. Beiderseits der Ebene 60 geht der Außenmantel 38 des Stutzens 34 gemäß Fig. 4 ohne Schulter unmittelbar in den Mantel 36 über. Beiderseits der Ebene 61 geht der Mantel 38 in die Stirnwand 33 und dann in den Außenmantel 32 über. Die Wände 32, 33 erstrecken sich nur über einen Teil des Spenderumfanges, so daß zwei radial nach außen vorstehende Queransätze 53 gebildet sind, welche gemeinsam mit dem Mantel 32 die radial äußersten Bereiche des Spenders 1 bilden. Die Außenseiten der Übergangswände 33 bilden daher konkave Handhaben 35, welche radial außerhalb der Handhabe 29 liegen. In der Endstellung liegt der Mantel 36 im Abstand vom Boden 27.

Beim Schließen oder Montieren des Gehäuses 25, 31 braucht lediglich der Mantel 36 zwischen die Mäntel 21, 26 in Richtung 13 eingeführt zu werden, ohne daß dabei der Körper 6 vom Körper 5 berührt wird. Der Stutzen 34 weist mit Radialabstand innerhalb des äußeren Stutzenmantels 38 einen inneren Stutzenmantel 37 auf, der in Richtung 13 über den Mantel 32 vorsteht, jedoch um mehr als seinen Durchmesser gegenüber dem freien Ende des Mantels 36 zurückversetzt ist. Die stromabwärtigen Enden der Mäntel 37, 38 gehen in einer Ringzone einteilig ineinander über und begrenzen dort die Öffnung 9, während der Mantel 37 den Kanal 8 begrenzt. Am Außenumfang kann das Rohr 37 Rippen 39 aufweisen, die in Axialebenen 58 liegen, welche einen Winkel von 45° zu den Ebenen 60, 61 einnehmen. Diese Rippen 39 schließen an den Mantel 38 nicht an.

Zur Öffnung der Folie 16 ist das Ende des Rohrs 37 ein konisches Werkzeug 40 mit abgestumpfter Spitze 41, die in divergierende und in den Ebenen 60, 61 liegende Stege 42 übergeht. Zwischen diesen Trennstegen 42 liegt jeweils eine Öffnung eines Kanaleinlasses 43.

Der Mantel 36 bzw. der Mantel 26 bildet eine radial nachgebende Feder 45, um Gegennocken 48 des Körpers 5 in Eingriff mit Anschlagnocken 47 des Körpers 4 bringen zu können, welche eine Abzugsicherung bilden. Die Nocken 47 liegen an den stromabwärtigen Enden der axialen Schlitze 46, welche zwischen den Mänteln 21, 26 auch den Boden 27 durchsetzen. Die acht Nocken 47 haben Bogenabstände voneinander, welche größer als ihr Bogenwinkel um die Achse 10 ist. Die zwei symmetrisch zur Ebene 61 beiderseits der Ebene 60 liegenden Nocken 48 haben jeweils um die Achse 10 eine so große Bogenerstrekkung, daß jeder Nocken 48 nur mit zwei Nocken 47 in Eingriff stehen kann.

Bei symmetrischer Drehausrichtung des Nockens 48 auf diese beiden Nocken 47 ist der Körper 5 allenfalls unter sehr großer Kraft bzw. durch Zerstörung vom Körper 4 abzuziehen, während bei symmetrischer Ausrichtung des Nockens 48 auf einen einzigen Nocken 47 diese Kraft wesentlich geringer ist und ein zerstörungsfreies Öffnen des Gehäuses 24, 31 erlauben kann. Die Nocken 48 schließen an das freie Ende des Mantels 36 an und bilden federnde Schnappnocken, welche bei der Montage die Nocken 47 zunächst federnd überlaufen. Die Nocken 47 stehen über den Innenumfang des Mantels 46 und die Nocken 48 über den Außenumfang des Mantels 36 vor.

Die Nocken 48 können auch in die Schlitze 46 eingreifen und dadurch eine formschlüssige Drehsicherung für die Körper 4, 5 bilden. In der Ruhelage liegen die Nocken 47, 48 als Abzugsicherung aneinander an. Auch der Speicherkörper 6 kann nach Trennung der Körper 4, 5 aus seiner Halterung 63 gelöst werden.

Zur Originalitätssicherung und auch zur Verhinderung unbeabsichtigter Betätigung ist für die Ruhelage eine Sperre 49 vorgesehen, welche das Trennen und auch das gegenseitige Verdrehen der Gehäuseteile 4, 5 erlaubt. Das einzige Sperrglied 52 liegt gemäß Fig. 3 an der Außenseite einer Flanke eines der trapezförmigen Ansätze 53 und schließt über ein Sollbruchglied 51 oder ein Gelenk einteilig an die Endfläche des Mantels 32 dieses Ansatzes 53 an.

Das Sperrglied 52 bildet dadurch ein Distanzglied, welches nur im Abstand vom Glied 51 an der Endfläche des Mantels 26 anliegt und gegenüber dem Körper 5 nur durch das Glied 51 abgestützt ist. Das Glied 52 ist ein zweiarmiger Hebel, dessen vom Mantel 26 entfernter Arm niedergedrückt werden kann, so daß sein Sperrarm gemäß Fig. 3 aus dem Bewegungsbereich des Mantels 26 geschwenkt werden kann. Das freie Ende des Sperrarmes bildet eine Handhabe 64 und kann am Außenumfang des Mantels 36 so anliegen, daß es mit einem Finger untergriffen und aus dem Bereich des Mantels 26 herausgezogen werden kann. Nach einer genügend großen Schwenkung kann das Glied 51 auch abscheren und dadurch das Glied 52 vollständig vom Spender 1 getrennt werden. Das Glied 52 kann während des Arbeitshubes aber auch am Mantel 36 gleiten.

Ferner ist das Glied 52 so angeordnet, daß sein Glied 51 bei einer entsprechend hohen Axialbelastung durch den Mantel 26 reißt und so die Sperre 59 überwunden ist. Dadurch ist eine Druckpunktsteuerung 50 geschaffen, welche bis zur Überwindung des Gliedes 51 die erhöhte axiale Druckkraft benötigt und dann schlagartig die wesentlich leichtgängigere Verschiebung der Körper 4, 5 erlaubt. Dies kann nicht nur zur schnellen Öffnung des Verschlusses 16, sondern auch zweckmäßig sein, wenn das Medium aus der Kammer 24 mit einer Pumpe gefördert wird, die mit der Axialbewegung betätigt wird. Beim Arbeits- bzw. Öffnungshub durchsticht die Spitze 41 den Verschluß 16, wonach die Rippe 39 mit ihren Endkanten 54 den Verschluß 16 weiter bis zum Mantel 18 aufschlitzen und die Spitze 41 den Boden 17 ggf. geringfügig nach unten drückt, wodurch die Kammer 24 eine für die Austragströmung günstigere Form erhalten kann und die Kompaktlagerung unterstützt wird.

Die vier Sicherungsglieder 63 liegen in Axialebenen 59, welche gegenüber den Ebenen 58, 60, 61 um die Achse 10 verdreht sind. In jeder dieser Ebenen 59 liegen auch zwei der Nocken 47 und der Vertiefungen oder Öffnungen 46.

Ist der Stutzen 34 nach dem Öffnen des Verschlusses 16 in eine Nasenöffnung eingeführt, so kann die Luft ventilfrei vom Einlaß 46, 56 bis zum Auslaß 9 in die Nase eingeatmet werden, wobei sie ab der Kammer 24 das Medium mitnimmt.

Die Flankenstege des Außenmantels 32 jedes Ansatzes 53 divergieren zur Ebene 61 spitzwinklig und gehen tangential in den Mantel 36 über, so daß sie im Bereich der Ebene 61 bzw. gemäß Fig. 3 im Bereich der Nocken 48 keinen Außenmantel bilden. Der Körper 4 bzw. Mantel 26 steht gemäß Fig. 3 nur zwischen den Ansätzen 53 bzw. im Bereich der Nocken 48 über die Außenkontur des Körpers 5 vor, während die Ansätze 53 den Mantel 26 bzw. den Gesamtkörper 4 abdecken.

Bei einer anderen Ausbildung des Spenders, z.B. mit Pumpe, kann die genannte Einlaßströmung auch direkt von der Kammer 24, beispielsweise einer Druckkammer, unter Druck in den Einlaß 43 gerichtet sein und allein das fließfähige, z.B. flüssige, Medium enthalten. Der Mantel 21 begrenzt einen durchgehend offenen Innenraum, welcher die Aufnahme 62 für den in der Halterung 63 gesicherten Speicher 7 bildet. Die Mäntel 21, 26 sind länger als der Mantel 36. Von allen Mänteln des Spenders ist der Mantel 37 der längste.

Zur Einbeziehung der Merkmale und Wirkungen in die vorliegende Erfindung wird auf die DE-OS 197 04 849 sowie auf die deutsche Patentanmeldung 198 31 525.2 Bezug genommen. Die dargestellten Größenverhältnisse sind besonders günstig. Die Eigenschaften und Wirkungen können genau oder nur im wesentlichen bzw. etwa wie beschrieben vorgesehen sein und je nach den Erfordernissen auch stärker davon abweichen.

## Patentansprüche

1. Spender für Medien mit zwei Einheiten (2, 3) und Grundkörpern (4, 5), nämlich einer ersten Einheit (2) mit einem ersten Grundkörper (4) und einer zweiten Einheit (3) mit einem zweiten Grundkörper (5), wobei die zweite Einheit (3) mit einer Kompaktlagerung gegenüber der ersten Einheit (2) aus einer Ruhelage in eine Endlage über einen Arbeitshub bewegbar ist, **dadurch gekennzeichnet, dass** die Kompaktlagerung drei Gehäusemäntel (21, 26, 36) aufweist, von denen zwei (26, 36) unter Bildung eines Laufmantels (36) und eines Führungsmantels (26) zusammenwirken und der weitere Führungsmantel (21) in einer Halterung (63) einen wenigstens eine Austragdosis des Mediums dicht aufnehmenden Speicher (7) umgibt, dass beide Führungsmäntel (21, 26) an dem ersten Grundkörper (4) angeformt sind und der Laufmantel (36) an dem zweiten Grundkörper (5), dass der Laufmantel (36) der Kompaktlagerung am Außenumfang von einem Außenmantel (32) des zweiten Grundkörpers (5) umgeben ist und dass eine den Außenmantel (32) und den Laufmantel (36) verbindende Endwand (33) des zweiten Grundkörpers (5) eine Betätigungshandhabe (35) der zweiten Einheit (3) bildet.

2. Spender nach Anspruch 1, **dadurch gekennzeichnet, daß** ein Laufmantel (36) der Kompaktlagerung die Halterung (63) permanent umgibt, und daß vorzugsweise mindestens einer der Führungsmäntel (21, 26) länger als der einzige zwischen sie eingreifende Laufmantel (36) ist.

3. Spender nach Anspruch 1 oder 2, **dadurch gekennzeichnet, daß** eine gegenseitige Arretierung für die Einheiten (2, 3) mit Arretiergliedem (47, 48) vorgesehen ist.

4. Spender nach Anspruch 3, **dadurch gekennzeichnet, daß** insbesondere die Arretierglieder (47, 48) am Innenumfang eines äußeren Führungsmantels (26) und einem Laufmantel (36) vorgesehen sind, und daß vorzugsweise einer der Gehäuse-Mäntel (26) im Bereich der Arretierglieder (47, 48) mit einer Vertiefung, wie einem Durchbruch (46), versehen ist.

5. Spender nach Anspruch 3 oder 4, **dadurch gekennzeichnet, daß** die Arretierung Arretierglieder (47, 48) enthält, die an der ersten Einheit (2) über den Umfang beabstandet verteilte Anschlagnocken (47) sowie an der zweiten Einheit (3) einen Gegennocken (48) umfassen, der sich über einen Bogenwinkel erstreckt, welcher größer als der innere bzw. äußere Bogenabstand zwischen den benachbarten Anschlagnocken (47) ist, und daß vorzugsweise die Einheiten (2, 3) relativ zueinander verdrehbar sind, wobei in einer ersten Drehstellung die Abzugsicherung gegen eine höhere Abzugkraft als in einer zweiten Drehstellung sichert.

6. Spender nach einem der vorhergehenden Ansprüche, **dadurch gekennzeichnet, daß** über einen Laufmantel (36) der Kompaktlagerung mindestens ein Queransatz (53) radial nach außen vorsteht, der nur über einen Teilumfang an den Laufmantel (36) tangential anschließt.

7. Spender nach einem der vorhergehenden Ansprüche, **dadurch gekennzeichnet, daß** die Einheiten (2, 3) mit einer der Kompaktlagerung zugeordneten Schnappverbindung aus federnden Schnappgliedem (47, 48) ineinander greifen, daß insbesondere ein an der ersten Einheit (2) vorgesehenes Schnappglied (47) mit Abstand von bzw mittig zwischen den Enden von Führungsmänteln (21, 26) liegt.

8. Spender nach einem der vorhergehenden Ansprüche, **dadurch gekennzeichnet, daß** Führungsmäntel (21, 26) der Kompaktiagerung von einer Endwand (27) des ersten Grundkörpers (4) frei abstehen und mit dieser Endwand (27) über die gesamte Länge des ersten Grundkörpers (4) reichen, daß an der Außenseite der Endwand (27) eine Betätigungshandhabe (29) gebildet ist.

9. Spender nach einem der vorhergehenden Ansprüche, **dadurch gekennzeichnet, daß** die an die Kompaktlagerung wenigstens annähemd anschließende Halterung (63) innerhalb des Außenumfanges eines inneren Führungsmantels (21) liegt.

10. Spender nach einem der vorhergehenden Ansprüche, **dadurch** gekennzheichnet, daß der Außenmantel (32) nur über einen Teilumfang des Laufmantels (36) reicht, und daß vorzugsweise der Außenmantel (32) in Umfangsrichtung in den Laufmantel (36) übergeht.

11. Spender nach einem der vorhergehenden Ansprüche, **dadurch gekennzeichnet, daß** der den Laufmantel (36) der Kompaktiagerung umgebende Außenmantel (32) kürzer als der Laufmantel (36) ist, daß insbesondere der Außenmantel (32) in der verkürzten Endstellung des Spenders (1) an einem äußeren Führungsmantel (26) anschlägt, und daß vorzugsweise eine den Außenmantel (32) und den Laufmantel (36) verbindende Endwand (33) des zweiten Grundkörpers (5) eine Betätigungshandhabe (35) der zweiten Einheit (3) bildet.

12. Spender nach einem der vorhergehenden Ansprüche, **dadurch gekennzeichnet, daß** die Einheiten (2, 3) in der Ruhelage mit einer lösbaren Sperre (49) gegen Bewegungen der Kompaktlagerung in Richtung zur Endlage formschlüssig gesichert sind, daß insbesondere die Sperre (49) ein die Endfläche eines äußeren Führungsmantels (26) blockierendes Sperrglied (52) umfaßt, das radial außerhalb eines Laufmantels (36) liegt und an einem Außenmantel (32) schwenkbar bzw. abreißbar befestigt ist, und daß vorzugsweise das Sperrglied (52) nur entlang einer Flanke eines Queransatzes (53) vorgesehen ist.

13. Spender nach einem der vorhergehenden Ansprüche, **dadurch gekennzeichnet, daß** ein Medienauslaß (9) über die Kompakdagerung gemeinsam mit der zweiten Einheit (3) bewegbar ist, daß insbesondere mindestens eine der Einheiten (2, 3) in der Endlage achssymmetrisch ausgebildet ist, und daß vorzugsweise die zweite Einheit (3) einen Dom (37) zur Öffnung eines Speicherverschlusses (16) aufweist sowie mit der ersten Einheit (2) Kanalwege (55) für die in den Speicher (7) gerichtete Einlaßströmung begrenzt.

14. Spender nach einem der vorhergehenden Ansprüche, **dadurch gekennzeichnet, daß** mindestens eine Einheit (2, 3) einschließlich der zugehörigen Lagerglieder (21, 26 bzw. 36, 37) der Kompaktlagerung ausschließlich aus dem zugehörigen einteiligen Grundkörper (4, 5) besteht.

15. Spender nach einem der vorhergehenden Ansprüche, **dadurch gekennzeichnet, daß** der zweite Grundkörper (5) einen frei vorstehenden Austragstutzen (34), wie einen Nasaistutzen, mit zwei im Abstand ineinander liegenden Stutzenmändeln (37, 38) bildet.

16. Spender nach einem der vorhergehenden Ansprüche, **dadurch gekennzeichnet, daß** der Spender (1) mit der Kompaktlagerung zum Austrag biologischer Wirkstoffe, wie pharmazeutischer Hilfsstoffe, ausgebildet ist, daß insbesondere die Wirkstoffe Peptide, Proteine und/oder Hormone sind, und daß vorzugsweise die Wirkstoffe wenigstens teilweise pulverförmig sind.

17. Spender nach einem der vorhergehenden Ansprüche, **dadurch gekennzeichnet, daß** der Spender (1) mit der Kompakttagerung zum Austrag pharmakokinetischer Adjuvantien ausgebildet ist, daß insbesondere das Adjuvans ein Freundsches Adjuvans umfaßt, und daß vorzugsweise das Adjuvans zur Verstärkung der Antwort des Immunsystems auf ein Arzneimittel, wie ein Antigen, diesem Arzneimittel beigemischt ist.

18. Spender nach einem der vorhergehenden Ansprüche, **dadurch gekennzeichnet, daß** die Betätigungshandhaben (35) konkav ausgebildet sind.

## Revendications

1. Distributeur de substances avec deux unités (2, 3) et deux corps de base (4, 5), c'est-à-dire une première unité (2) avec un premier corps de base (4) et une deuxième unité (3) avec un deuxième corps de base (5), sachant que la deuxième unité (3) est déplaçable avec un logement compact par rapport à la première unité (2) à partir d'une position de repos dans une position terminale par une course de travail, **caractérisé en ce que** le logement compact présente trois enveloppes de boîtier (21, 26, 36), dont deux (26, 36) agissent conjointement en formant une enveloppe de glissement (36) et une enveloppe de guidage (26), et l'autre enveloppe de guidage (21) dans un support (63) entoure un réservoir (7) logeant de manière étanche au moins une dose de décharge de substance, **en ce que** les deux enveloppes de guidage (21, 26) sont ajoutées par moulage au premier corps de base (4) et l'enveloppe de glissement (36) au deuxième corps de base (5), **en ce que** l'enveloppe de glissement (36) du logement compact est entouré à la circonférence extérieure par une enveloppe extérieure (32) du deuxième corps de base (5) et **en ce qu'**une paroi terminale (33) du deuxième corps de base (5), raccordant l'enveloppe extérieure (32) et l'enveloppe de glissement (36), forme une manette d'actionnement (35) de la deuxième unité (3).

2. Distributeur d'après la revendication 1, **caractérisé en ce qu'**une enveloppe de glissement (36) du logement compact entoure de manière permanente le support (63), et **en ce que** de préférence au moins une des enveloppes de guidage (21, 26) est plus longue que l'unique enveloppe de glissement (36) qui s'engage entre elles.

3. Distributeur d'après la revendication 1 ou 2, **caractérisé en ce qu'**on prévoit un mécanisme d'arrêt réciproque pour les unités (2, 3) avec des éléments d'arrêt (47, 48).

4. Distributeur d'après la revendication 3, **caractérisé en ce que** notamment les éléments d'arrêt (47, 48) sont prévus à la circonférence intérieure d'une enveloppe extérieure de guidage (26) et sur une enveloppe de glissement (36), et **en ce que** de préférence une des enveloppes de boîtier (26) est muni d'un creux, comme une ouverture (46), dans le domaine des éléments d'arrêt (47, 48).

5. Distributeur d'après la revendication 3 ou 4, **caractérisé en ce que** le mécanisme d'arrêt comprend des éléments d'arrêt (47, 48), qui comprennent des cames d'arrêt (47) distribuées avec un écart entre elles sur la première unité (2) à la circonférence, ainsi qu'une came accessoire (48) sur la deuxième unité (3), qui s'étend sur un angle d'enroulement plus grand que l'écart d'enroulement intérieur ou encore extérieur entre les cames d'arrêt (47) limitrophes, et **en ce que** de préférence les unités (2, 3) peuvent être déplacées angulairement l'une par rapport à l'autre, sachant que dans une première position angulaire l'assurage contre un détachement protège contre une force de traction plus haute que dans une deuxième position angulaire.

6. Distributeur d'après une des revendications précédentes, **caractérisé en ce qu'**au-delà d'une enveloppe de glissement (36) du logement compact au moins une appendice transversale (53) saille radialement vers l'extérieur et il fait suite à l'enveloppe de glissement (36) de façon tangentielle uniquement sur une partie de la circonférence.

7. Distributeur d'après une des revendications précédentes, **caractérisé en ce que** les unités (2, 3) font prise l'une sur l'autre par un raccord à déclic, associé au logement compact, composé d'éléments à déclic (47, 48) élastiques, **en ce que** notamment un élément à déclic (47) prévu sur la première unité (2) se trouve à une certaine distance des ou encore au milieu entre les extrémités des enveloppes de guidage (21, 26).

8. Distributeur d'après une des revendications précédentes, **caractérisé en ce que** des enveloppes de guidage (21, 26) du logement compact saillent librement d'une paroi terminale (27) du premier corps de base (4) et s'étendent avec cette paroi terminale (27) sur toute la longueur du premier corps de base (4), **en ce que** à la face extérieure de la paroi terminale (27) est formée une manette d'actionnement (29).

9. Distributeur d'après une des revendications précédentes, **caractérisé en ce que** le support (63), qui fait suite au moins approximativement au logement compact, se trouve à l'intérieur de la circonférence extérieure d'une enveloppe intérieure de guidage (21).

10. Distributeur d'après une des revendications précédentes, **caractérisé en ce que** l'enveloppe extérieure (32) s'étend seulement sur une partie de la circonférence de l'enveloppe de glissement (36), et **en ce que** de préférence en direction de circonférence l'enveloppe extérieure (32) devient l'enveloppe de glissement (36).

11. Distributeur d'après une des revendications précédentes, **caractérisé en ce que** l'enveloppe extérieure (32) qui entoure l'enveloppe de glissement (36) du logement compact est plus courte que l'enveloppe de glissement (36), **en ce que** notamment l'enveloppe extérieure (32) dans la position terminale raccourcie du distributeur (1) aboutit à une enveloppe extérieure de guidage (26), et **en ce que** de préférence une paroi terminale (33) du deuxième corps de base (5), qui raccorde l'enveloppe extérieure (32) et l'enveloppe de glissement (36), forme une manette d'actionnement (35) de la deuxième unité (3).

12. Distributeur d'après une des revendications précédentes, **caractérisé en ce qu'**en position de repos les unités (2, 3) sont assurées à engagement positif contre des mouvements du logement compact en direction vers la position terminale par un verrouillage (49) amovible, **en ce que** notamment le verrouillage (49) comprend un élément de blocage (52), bloquant la surface terminale d'une enveloppe extérieure de guidage (26), qui se trouve radialement à l'extérieur d'une enveloppe de glissement (36) et qui est attaché à une enveloppe extérieure (32) de manière à pouvoir être pivoté ou encore arraché, et **en ce que** de préférence l'élément de blocage (52) n'est prévu que le long d'un flanc d'une appendice transversale (53).

13. Distributeur d'après une des revendications précédentes, **caractérisé en ce qu'**un orifice de sortie de substance (9) est déplaçable au-dessus du logement compact conjointement avec la deuxième unité (3), **en ce que** notamment au moins une des unités (2, 3) en position terminale est réalisée de manière symétrique par rapport à une droite, et **en ce que** de préférence la deuxième unité (3) présente un poinçon (37) pour l'ouverture de la fermeture de réservoir (16) ainsi qu'elle délimite avec la première unité (2) des voies de conduit (55) pour le courant d'entrée orienté à l'intérieur du réservoir (7).

14. Distributeur d'après une des revendications précédentes, **caractérisé en ce qu'**au moins une unité (2, 3) y compris les éléments de logement (21, 26 ou encore 36, 37) correspondants du logement compact est constitué uniquement du corps de base (4, 5) monobloc correspondant.

15. Distributeur d'après une des revendications précédentes, **caractérisé en ce que** le deuxième corps de base (5) forme une tubulure de décharge (34) librement saillante, comme une tubulure nasale, avec deux enveloppes de tubulure (37, 38) situées à une certaine distance l'une à l'intérieur de l'autre.

16. Distributeur d'après une des revendications précédentes, **caractérisé en ce que** le distributeur (1) avec le logement compact est réalisé pour la décharge de substances actives biologiques, telles que des agents accessoires pharmaceutiques, **en ce que** notamment les substances actives sont des peptides, des protéines et/ou des hormones, et **en ce que** de préférence les substances actives se présentent au moins en partie sous forme de poudre.

17. Distributeur d'après une des revendications précédentes, **caractérisé en ce que** le distributeur (1) avec le logement compact est réalisé pour la décharge d'adjuvants pharmacocinétiques, **en ce que** notamment l'adjuvant comprend un adjuvant de Freund, et **en ce que** de préférence l'adjuvant est ajouté à un médicament pour affermir la réponse du système immunitaire à ce médicament, pouvant être un antigène.

18. Distributeur d'après une des revendications précédentes, **caractérisé en ce que** les manettes d'actionnement (35) sont réalisées de manière concave.

## Claims

1. A dispenser for media including two units (2, 3) and base bodies (4, 5), namely a first unit (2) including a first base body (4) and a second unit (3) including a second base body (5), the second unit (3) with a compact mount being movable relative to the first unit (2) from a resting position into an end position via a working stroke, **characterized in that** the compact mount comprises three housing shells (21, 26, 36), two of which (26, 26) cooperate in forming a runner shell (36) and a guide shell (26) whilst the further guide shell (21) in a support (63) surrounds a sealed reservoir (7) containing at least one dosed delivery of the medium, that both guide shells (21, 26) are molded on the first base body (4) and the runner shell (36) on the second base body (5), that the runner shell (36) of the compact mount is surrounded at its outer circumference by an outer shell (32) of the second base body (5) and **in that** an end wall (33) of the second base body (5) connecting the outer shell (32) and the runner shell (36) forms an actuating finger hold (35) of the second unit (3).

2. The dispenser as set forth in claim 1, **characterized in that** the runner shell (36) surrounds the support (63) at all times, and that preferably at least one of the guide shells (21, 26) is longer than the sole runner shell (36) engaging between them.

3. The dispenser as set forth in claim 1 or 2, **characterized in that** a mutual arresting device is provided for the units (2, 3) by arresting members (47, 48).

4. The dispenser as set forth in claim 3, **characterized in that** in particular the arresting members (47, 48) are provided on the inner circumference of an outer guide shell (26) and on a runner shell (36) and that preferably one of the housing shells (26) is provided in the region of the arresting members (47, 48) with a recess such as a breakthrough (46).

5. The dispenser as set forth in claim 3 or 4, **characterized in that** the arresting device contains arresting members (47, 48) comprising stoppers (47) distributed spaced away from each other about the circumference of the first unit (2) as well as a cam (48) on the second unit (3) extending over an angle of an arc which is greater than the inner or outer arc spacing between adjacent stoppers (47) and **in that** preferably the units (2, 3) are rotatable relative to each other, the captive lock preventing removal at a force higher in a first rotated position than in a second rotated position.

6. The dispenser as set forth in any of the preceding claims, **characterized in that** at least one transverse appendage (53) protrudes radially outwards beyond the runner shell (36) of the compact mount, the transverse appendage (53) adjoining the runner shell (36) tangentially only via part of the circumference.

7. The dispenser as set forth in any of the preceding claims, **characterized in that** the units (2, 3) interengage by a snap-action connection of springy snap-action members (47, 48) assigned to the compact mount, more particularly a snap-action member (47) provided on the first unit (2) being located spaced away from or in the middle between the ends of the guide shells (21, 26).

8. The dispenser as set forth in any of the preceding claims, **characterized in that** the guide shells (21, 26) of the compact mount freely protrude from an end wall (27) of the first base body (4) and extend with this end wall (27) over the full length in the base body (4), and that an actuating finger rest (29) is formed at the outer side of the end wall (27).

9. The dispenser as set forth in any of the preceding claims, **characterized in that** the support (63) adjoining the compact mount at least approximately is located within the outer circumference of an inner guide shell (21).

10. The dispenser as set forth in any of the preceding claims, **characterized in that** the outer shell (32) extends only over part of the circumference of the runner shell (36) and that preferably the outer shell (32) translates circumferentially into the runner shell (36).

11. The dispenser as set forth in any of the preceding claims, **characterized in that** the outer shell (32) surrounding the runner shell (36) of the compact mount is shorter than the runner shell (36), more particularly the outer shell (32) coming up against an outer guide shell (26) in the shortened end position of the dispenser (1) and that preferably an end wall (33) of the second base body (5) connects the outer shell (32) and the runner shell (36) forming an actuating finger rest (35) of the second unit (3).

12. The dispenser as set forth in any of the preceding claims, **characterized in that** the units (2, 3) are positively locked in the resting position by a defeatable lock (49) against motion of the compact mount in the direction of the end position, more particularly the lock (49) comprising a locking member (52) blocking the end face of the first guide shell (26), the locking member (52) being located radially outside of the runner shell (36) and secured swivably or removably to an outer shell (32) and that preferably the locking member (52) is provided only along the flank of a transverse appendage (53).

13. The dispenser as set forth in any of the preceding claims, **characterized in that** a medium orifice (9) is movable together with the second unit (3) via the compact mount, more particularly at least one of the units (2, 3) being configured axially symmetrical in the end position and preferably the second unit (3) comprising a spike (37) for opening a reservoir closure (16) as well as defining with the first unit (2) the passageways (55) for the inlet flow directed into the reservoir (7).

14. The dispenser as set forth in any of the preceding claims, **characterized in that** at least one unit (2, 3) including the corresponding mounting members (21, 26 and 36, 37 respectively) of the compact mount consists exclusively of the corresponding integral base bodies (4, 5).

15. The dispenser as set forth in any of the preceding claims, **characterized in that** the second base body (5) forms a freely protruding discharge nozzle (34) such as a nasal nozzle having two nested nozzle shells (37, 38) spaced away from each other.

16. The dispenser as set forth in any of the preceding claims, **characterized in that** the dispenser (1) with the compact mount is configured for delivering biological active substances, such as pharmaceutical additives, more particularly the active substances being peptides, proteins and/or hormones and that preferably the active substances are powdery at least in part.

17. The dispenser as set forth in any of the preceding claims, **characterized in that** the dispenser (1) with the compact mount is configured for delivering pharmacokinetic adjuvants, more particularly the adjuvant comprising a Freund adjuvant and that preferably the adjuvant is added to a drug such as an antigen, to enhance the response of the immune system.

18. The dispenser as set forth in any of the preceding claims, **characterized in that** the actuating finger rests (35) are configured concave.
